# EUROPEAN PATENT APPLICATION

(11) **EP 4 582 783 A1**
(43) Date of publication of application: **09.07.2025**
(21) Application number: 25150238.1
(22) Date of filing: 03.01.2025
(51) Int. Cl.: G01M 3/20, G01M 3/22

(54) **SYSTEM AND METHOD FOR TRAINING A GAS DETECTION MODEL**

(30) Priority: 05.01.2024 US 202418405190
(71) Applicant: Honeywell International Inc., Charlotte, NC 28202 (US)
(72) Inventor: KASTELEIN, Bas, Charlotte, 28202 (US); TRENCHARD, Andrew J., Charlotte, 28202 (US); SHEN, Quan, Charlotte, 28202 (US)
(74) Representative: Haseltine Lake Kempner LLP

(57) **Abstract**

A system and method for training a machine learning gas detection model that monitors gas sensors located at an industrial site. The system and method uses a digital twin arranged to execute simulations of gas leaks using a virtual representation of the physical industrial site and varying simulated wind patterns, gas leak locations and leak rates. The simulations executed by the digital twin train a machine learning gas detection model with time-series gas sensor responses for the simulated gas leaks executed by the digital twin. The trained gas detection model is used in a gas detection system to monitor for gas leaks at the physical industrial site.

## Description

### TECHNICAL FIELD

This disclosure relates to a system and method for training a machine learning gas detection model using simulations of an industrial site and its weather using a digital twin.

### BACKGROUND

Currently known solutions that detect, localize, and quantify gas leaks originating from gas handling and transport equipment typically use heuristic models to correlate detection of the gas leaks. Wind direction and windspeed are used in the heuristic models to triangulate the leak location and using a dynamic plume model, e.g., a Gaussian plume model, to detect and locate the gas leak to obtain a quantification of a leak rate. This leak detection can be used to warn the operators of an industrial plant or gas transport terminals of the leak. However, the heuristic model in practice has limits on the accuracy of leak location and quantification, due to the stochastic nature of atmospheric variables, such as for example, the windspeed and direction of the wind and the physics of the gas dispersion process assumed by the heuristic model.

Monitoring for leak detections near real-time with a continuous automated monitoring solution allows for a correlation of the processes that may cause the leak, providing the opportunity to change these processes to avoid leaks in the future. A machine learning model can be used to localize and quantify a leak from time-series data of gas concentrations, windspeed and wind direction together with other environmental variables such as humidity and temperature. Machine learning models, however, require extensive training in order to model a physical industrial site. Such training would need to be done through a series of controlled releases of gas using various rates of gas discharges at various locations and under varying wind conditions. However, such gas discharges for machine learning, even under controlled conditions, would be detrimental to the plant, plant employees, or not allowed by plant management and the surrounding community. The machine learning model could be trained while in an operational state in the field by feeding-back field measurements of actual found leaks through manual inspections. However, this approach is very labor intensive requiring entry of literally thousands or more of training data points into the machine learning model collected over an extended period of time.

It is therefore the object of the present disclosure to provide a system and method for training a machine learning gas detection model using simulations of an industrial site using a digital twin.

### SUMMARY

This disclosure relates to a system and method for training a machine learning gas detection model using simulations of an industrial site and its weather using a digital twin.

In a first embodiment, a system is disclosed for training a machine learning gas detection model used for monitoring an industrial site for gas leaks. The system comprises a gas sensor module containing data representing the predefined locations of gas sensors; a gas sensor response model that models the responses of the gas sensors; and a digital twin containing a virtual representation of the physical equipment located at the industrial site. The digital twin is arranged to execute simulations of gas leaks using the virtual representation of the industrial site using varying simulated wind patterns, gas leak locations and leak rates and data representing the gas sensor locations to generate simulated time-series gas sensor responses. The time-series gas sensor responses are used to train the machine learning gas detection model for detecting gas leaks at the industrial site.

In a second embodiment a method is disclosed for training a machine learning gas detection model used in monitoring an industrial site for gas leaks. The method comprises providing data representing the locations of gas sensors and a gas sensor response model that models the responses of the gas sensors. The method further comprises providing a digital twin containing a virtual representation of the physical equipment located at the industrial site. The digital twin is arranged to execute simulations of gas leaks using varying simulated wind patterns and varying gas leak locations and leak rates using data representing the gas sensor locations including simulated gas sensor responses to generate time-series gas sensor responses for the simulated gas leaks. The machine learning gas detection model is trained with the gas sensor responses.

In a third embodiment, a gas detection system executing in a plant server is disclosed used to monitor an industrial site for gas leaks. The plant server is communicatively coupled to a plurality of gas sensors and to at least one weather station and to a training system used to train the gas detection system The training system comprises a digital twin executing in the training system containing a virtual representation of the physical equipment located at the industrial site. A gas sensor module coupled to the digital twin contains data representing the locations of the plurality of gas sensors at the industrial site. A gas sensor response model that models the responses of the gas sensors and a machine learning gas detection model coupled to the digital twin. The machine learning gas detection model is trained with time-series gas sensor responses generated by simulations of virtual gas leaks run by the digital twin using varying simulated wind patterns and varying gas leak locations and leak rates using the virtual representations of the physical equipment at the industrial site and the data representing the locations of the plurality of gas sensors and the expected responses from the gas sensors provided by the gas sensor response model. The trained machine learning gas detection model is coupled to the plant server and to the gas detection system and used by the plant server to monitor the industrial site for gas leaks using the trained gas sensor responses.

Other technical features may be readily apparent to one skilled in the art from the following figures, descriptions, and claims.

### BRIEF DESCRIPTION OF THE DRAWINGS

For a more complete understanding of this disclosure, reference is now made to the following description, taken in conjunction with the accompanying drawings, in which:
FIG. 1 is an overhead arial view of an industrial site illustrating the placement and locations of gas sensors and weather stations according to the present disclosure;
FIG. 2 is an exemplary system used to monitor gas sensors and the weather stations and to locate and quantify gas leaks at an industrial site according to the present disclosure;
FIG. 3 is a diagram of the models executing in the digital twin according to the present disclosure;
FIG. 4 is an illustration of an example of a data preparation simulation for teaching the machine learning model according to the present disclosure;
FIG. 5 is an illustration of an example time-series dataset produced by the simulated data of the data preparation simulation of FIG. 4;
FIG. 6 is a scatter plot of the leak localization training simulation using the data of FIG. 5;
FIG. 7A is a block diagram of an exemplary method used to train a machine learning gas detection model according to the present disclosure; and
FIG. 7B is a block diagram of an exemplary method used by a machine learned gas detection model to monitor the physical industrial site and a method for continuous learning of the machine learned gas detection model according to the present disclosure.

### DETAILED DESCRIPTION

The figures discussed below, and the various embodiments used to describe the principles of the present invention in this patent document are by way of illustration only and should not be construed in any way to limit the scope of the invention. Those skilled in the art will understand that the principles of the invention may be implemented in any type of suitably arranged device or system

Various illustrative configurations of systems and their methods are known to detect and report the emission of gas from monitored sites for a variety of reasons. For example, a monitored industrial site may be an oil facility removing natural gas (and/or oil) from an underground reservoir that utilizes equipment (e.g., pumpjacks, holding tanks, valves, pipes, etc.) that requires maintenance. Intermittently or continuously, this equipment releases the gas into the atmosphere. Such a release is called a 'fugitive gas emission' that should be detected and reported so corrective action may be taken.

The present disclosure relates to a gas detection system comprised of a machine learning gas detection model that is trained using simulated data created by a running a gas dispersion model on a digital twin of the industrial site. The simulation uses a gas dispersion model, for example a Gaussian Plume or other model and a detector gas response function to compute simulated time-series detector responses for gas leaks of varied sizes at different leak locations at the industrial site and at different weather conditions. These simulated sensor responses and weather data are then used to train a machine learning gas detection model.

The trained machine learning gas detection model is then deployed in the gas detection system at the industrial site and tuned using the physical industrial site operational data. Actual time-series data of gas concentrations and weather data are input into the machined learned gas detection model along with any reported leak locations and leak rates from the gas sensors deployed at the industrial site.

Reported actual leak locations and leak rates obtained from field inspections are historized in a historian and retrieved along with the historic time-series data of gas concentrations and weather data. This retrieved historized data is input back into the digital twin to retrain the machine learning gas detection model. The restrained machine learned gas detection model is then redeployed back to the gas detection system

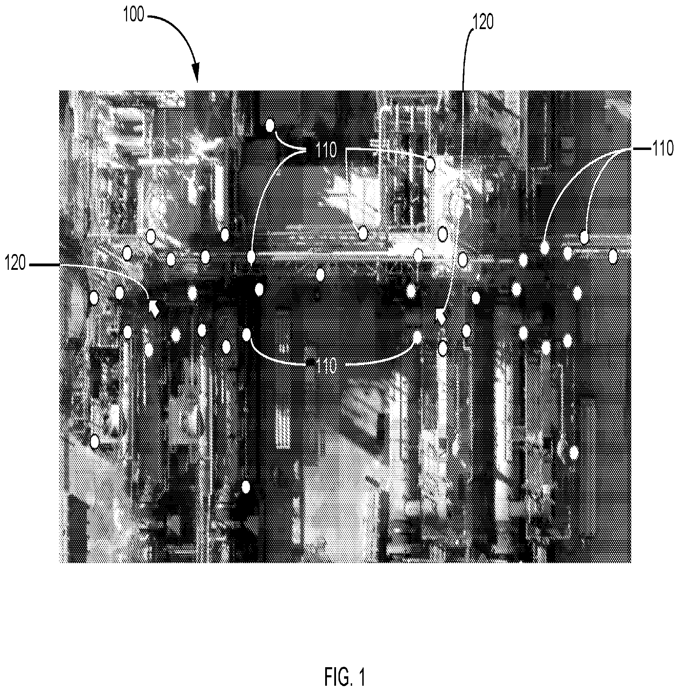

With reference to FIG. 1 an industrial site 100 is shown containing gas sensors 110 and weather stations 120. Multiple gas sensors 110 are geospatially distributed in around external area near assets of the industrial site 100, such as for example, a well-heads, vessels, pipelines, or other refining or chemical manufacturing assets that may be a potential leak source. One or more weather stations 120 are also located near or amongst the gas sensors 120. The weather stations 120 may be comprised of one or more anemometers and temperature and humidity sensors used to sense and report wind direction, wind speed, temperature, and humidity. The gas sensors 110 measure the gas concentration levels at a specific time intervals such as for example, between 2 and 30 seconds and the weather stations 120 also record the wind direction and wind speed at the corresponding gas sensor reading intervals.

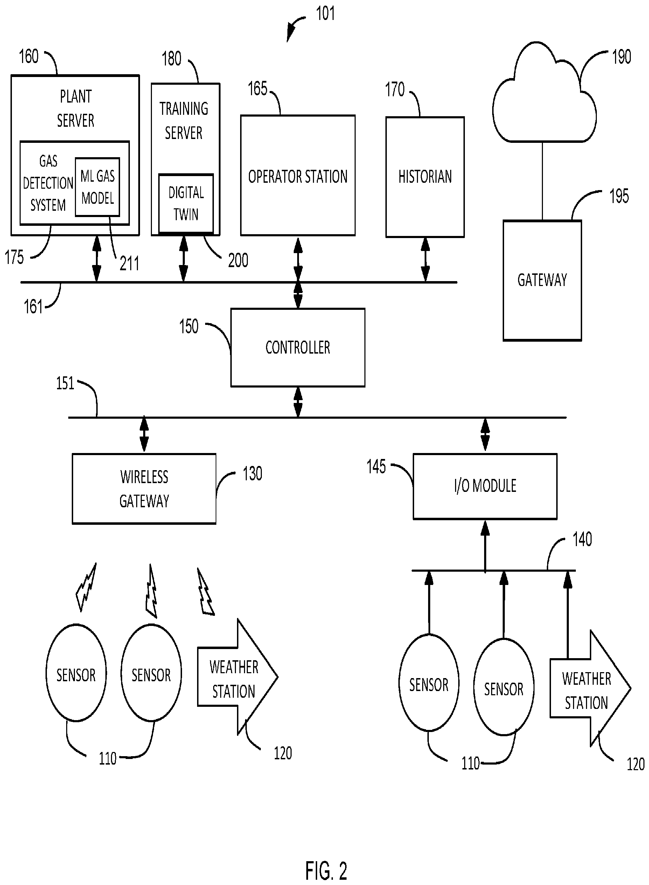

FIG. 2 illustrates an exemplary system 101 used to monitor the gas sensors 110 and the weather stations 120 that may be used for processing the information gathered to locate and quantify any gas emissions from the plant 100. At a device layer of the system 101 the gas sensors 110 and weather stations 120 are connected either to a wireless gateway 130 or a wired network 140 via an 1/0 module 145. More than one wireless gateway 130 may be deployed in the device layer. Each wireless gateway has a plurality of wireless gas sensors 110 and wireless weather stations 120 connected to the gateway 130. Similarly, more than one 1/0 module 145 may be used to connect to a plurality of wired gas sensors 110 and wired weather stations 120 using a wired network 140.

At a control layer, the system 101 may include one or more controllers 150 connected to the wireless gateway 130 and the 1/0 module 145 via a control network 151. The controller 150 can be used in the system 101 to perform various functions in order to control the data gathering process from the gas sensors 110 and weather stations 120. For example, the controller 150 may act as a supervisor to control the transfer of sensor data and weather data from weather data collected by the gateway 130 and 1/0 module 145. The controller 150 can also be used to provide diagnostic information to the system 101 of the operational health of the wireless gateway 130, 1/0 module 145 and the gas sensors 110 and weather stations 120.

The controller 150 transfers the collected data to a plant server 160 via a plant communication network 161. The plant server 160 denotes a computing device that executes data processing programs and applications including a gas detection system 175 that uses the pre-trained machine learning gas detection model 210 generated by the method of the present disclosure. The plant server 160 could represent any computing device running a **WINDOWS** operating system or other operating system. Note that while shown as being local within system 101, the functionality of the server 160 could be represented by a server or other computing device remotely located from the system 101. For instance, the functionality of the server 160 could be implemented in a remotely located computing device in a cloud 190 communicatively coupled to the system 101 via a gateway 195.

The system 101 further includes a training server 180 operatively connected to the plant communication network 161. The training server 180 denotes a computing device that executes data processing programs and applications including a digital twin 200 used to pre-train a machine learning gas detection model 210 using the method of the present disclosure. The training server 180 could represent any computing device running a **WINDOWS** operating system or other operating system. Note that while shown as being local within system 101, the functionality of the training server 180 may be represented by a server or other computing device remotely located from the system 101. For instance, the functionality of the server 180 could be implemented in a remotely located computing device in a cloud 190 communicatively coupled to the system 101 via a gateway 195.

Operator access to and interaction with the controller 150, plant server 160 and training server 180 and other components of the system 101 can occur via one or more operator consoles 165 connected to the plant communication network 161. Each operator console 165 could be used to provide information to an operator and receive information from an operator. For example, each operator console 165 could provide information identifying a current state of a plant process, such as the reported values of gas sensors 110 and weather data from the weather stations 120 and various displays associated with monitoring and detection of gas leaks at the plant. Each operator console 165 could also receive information affecting how the industrial process is controlled, such as by receiving setpoints or control modes for the gas sensors 110 and weather stations 120 that alters or affects how the controller 150 controls the system
101. Each operator console 165 includes any suitable structure for displaying information to and interacting with an operator. For example, each operator console 165 could represent a computing device running a **WINDOWS** operating system or other operating system

The plant operations layer of system 101 also includes at least one historian 170. The historian 170 represents a component that stores using memory storage devices information about the system 101. The historian 170 could, for instance, store information that is gathered by the gas sensors 110 and weather stations 120 for processing by plant server 160. The historian 170 includes any suitable structure for storing and facilitating retrieval of information. Although shown as a single component here, the historian 170 could be located elsewhere in the system 101, or multiple historians could be distributed in separate locations in the system 101 along the plant communication network 161.

Although FIG. 2 illustrates one example of a system 101, various changes may be made to FIG. 2. For example, the system 101 could include any number of sensors, actuators, controllers, networks, operator consoles, control rooms, historians, servers, wireless devices, and other components. Also, the makeup and arrangement of the system 101 in FIG. 2 are for illustration only. Components could be added, omitted, combined, further subdivided, or placed in any other suitable configuration according to particular needs. Further, particular functions have been described as being performed by particular components of the system 101. In general, control systems are highly configurable and can be configured in any suitable manner according to particular needs.

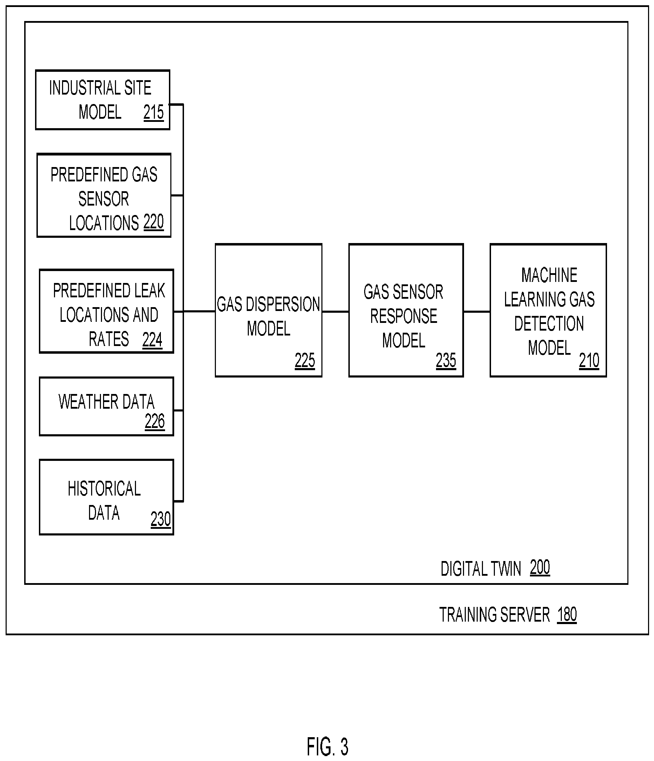

A diagram of an embodiment of the digital twin 200 of the present disclosure is shown in FIG. 3. In the exemplary embodiment, the digital twin 200 executes in the training server 180 to train a machine learning gas detection model 210. Within the framework of the present disclosure, the digital twin 200 is a virtual environment containing a parametrization of data corresponding to actual physical objects. The digital twin 200 contains the parameterization of data and information of the physical equipment and assets installed at the industrial site 100. The physical objects of the industrial site 100 may include equipment for various purposes, such as for example, elements or systems for the production of products, and/or systems for storage and transportation of the products manufactured by the industrial production process. Physical parameters of the equipment such as, length, shape and dimensions and operating characteristics are also parameterized as data for use by the digital twin 200.

The digital twin 200 is represented in the present embodiment as a software module executing in the training server 180. The digital twin 200 may also be represented by a separate hardware and software system implemented as part of a hardware and software complex that implements specialized software that allows for the processing of digital twin's requests that create new digital twins for transfer back to a training server to which it may be operationally connected.

In the present embodiment, the equipment and assets located at the industrial site are modeled and represented as data in an industrial site model 215 and input into the digital twin 200. Data representing predefined locations for a number of gas sensors 220, predefined leak locations and rates 224 and predefined weather data 226 are modeled and represented as data for use by the digital twin 200 in the simulation. The digital twin 200 uses the data representing the assets and equipment of the industrial site input by the industrial site model 215 and the predefined sensor locations from module 220, leak locations and leak rate data 224 and the predefined weather data 226 to run simulations using a gas dispersion model 225 and a gas sensor response model
235. The digital twin 200 uses the sensor response model 235 to compute simulated time-series gas sensor responses to simulated gas leaks at different leak rates at different leak locations, from the predefined gas sensor locations 220, predefined leak locations and rates 224 and at different weather conditions provided by the predefined weather data 226. The weather conditions include varying wind patterns of wind direction and speed.

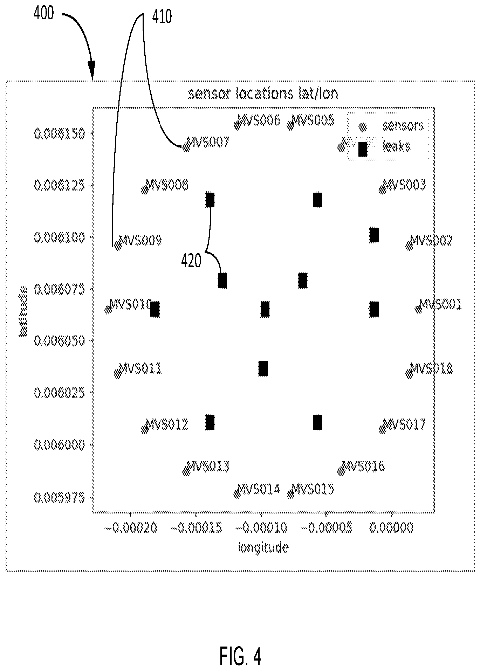

FIG. 4-6 illustrates an example embodiment of a training simulation 400 that may be run by the digital twin 200 for training the machine learning gas detection model 210. In FIG. 4, the data is prepared by locating simulated predefined gas sensors 410 in various locations using latitude and longitude coordinates. In the illustration of FIG. 4 the gas sensors 410 are arranged in a ring pattern for the simplicity of explanation of the data preparation of the method, however, the gas sensors 410 may also be arranged according to the latitude and longitude of the locations of the gas sensors 110 located at the industrial site 100. Each gas sensor 410 of the training simulation would generate data during a fixed time interval. In this example, every 5 seconds. Simulated weather data is generated on a frequency of every 5 seconds. The weather data for example may include general assumptions such as: a. the wind direction will be fixed for 1 minute; b. that the wind direction will change from Oto 360 degrees; c. that the wind speed will vary every 5 seconds; and d. that the wind speed may change between Oto 2.5 mph, to include a "O" or a no wind speed on a random basis. However, if weather data is known for the industrial site 100 then actual weather data may be used instead of the assumptions. The simulated locations 420 for gas leaks are specified in the simulation.

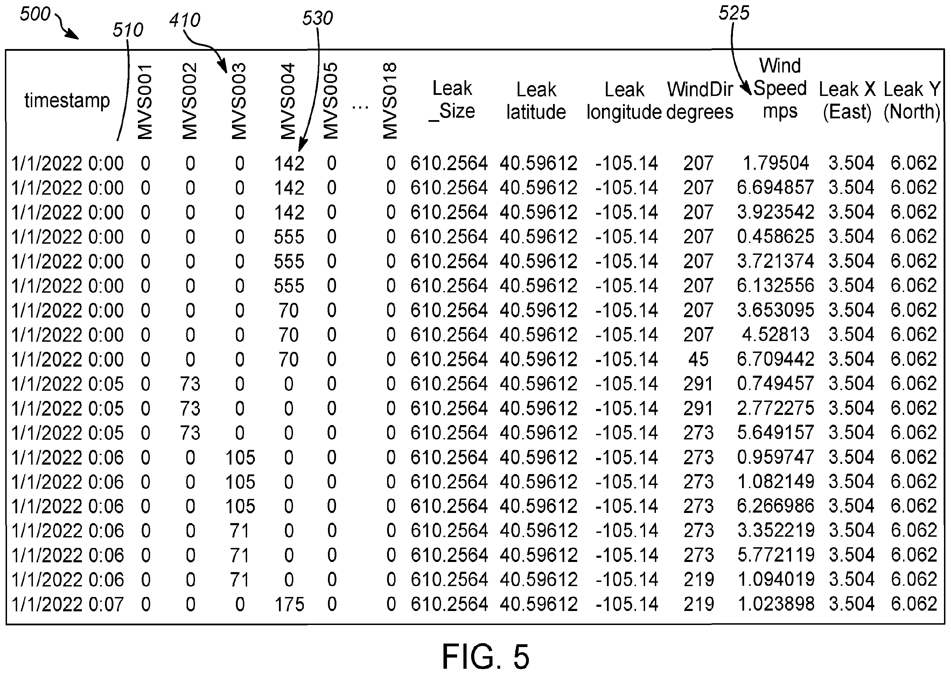

A dataset 500 is then constructed, as shown in FIG. 5 that combines the generated gas sensor readings 530 from each gas sensor 410, with the simulated wind variable data 525 along with a timestamp 510. For each timestamp, a gas sensor reading 530 would be registered for each individual gas sensor 410 in the dataset 500 based on input from the gas sensor response model 235. For example, for the time stamp 1/1/2022 0:00 gas sensor MVS004 registers a gas reading of 142 for a wind data 525 having a direction of 207 degrees at a wind speed of 1.79504 mph. Since the dataset will be used to train the machine learning gas detection model the leak size and true leak location is kept constant and only the wind speeds and directions are changed to simulate how the gas sensors 410 would react to a gas leak from a fixed point in varying wind conditions. A predicted leak coordinate in ax (east) and y (north) coordinate will show distance in meters from the latitude and longitude of the location of the leak.

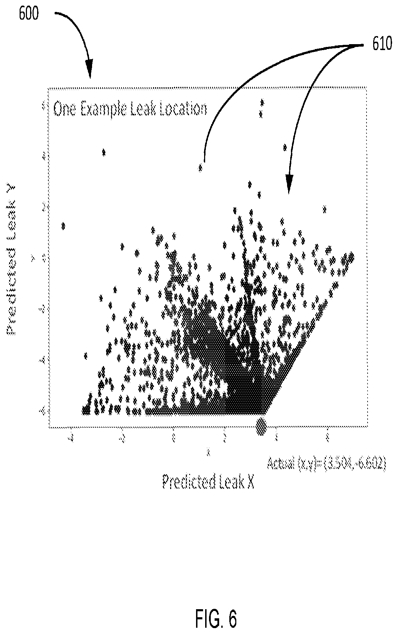

FIG. 6 illustrates a scatter plot 600 representing 5 second sample leak prediction estimates for the dataset example of FIG. 5 for the actual coordinates of the simulated gas leak is generated using the gas dispersion model 225. Each scatter point 610 representing on the scatter plot 500 a 5 second sample estimate. The simulated predictions points 610 from the dataset indicate radial concentrations ofleak predictions for the varying wind directions and speed. The data generated from running the simulated leak predictions are used to train the machine learning gas detection model 210.

The now trained machine learning gas detection model 210 is loaded into a gas leak detection system 175 running in plant server 160 as a machine learned gas detection model 211. Actual time-series data of the gas sensors 110 and weather stations 120 located at the site 100 and connected to system 101 are input to the gas leak detection system 175. The actual data gas concentrations from the sensors 110 and weather data from the weather stations 120 are analyzed by the machine learned gas detection model 211. The machine learned gas detection model 211 reports to the gas detection system the gas leak locations and gas leak locations of gas leaks based on the data simulations that the machine learned gas detection model was trained with the digital twin.

At the same time the actual leak locations and leak rates obtained from field inspections obtained from field inspections are recorded in the historian 170 along with the time-series data of concentrations of gas reported by the sensors 110 and weather data from weather stations 120.

The historized data from the historian 170 of actual found gas leaks and/or leak rates are fed-back to the digital twin 200. Leak location data and the actual time-series data from gas concentrations recorded by historian 170 are also fed-back to the digital twin 200 to provide a continuous-learning process that retrains the machine learning gas detection model 210 in the digital twin 200.

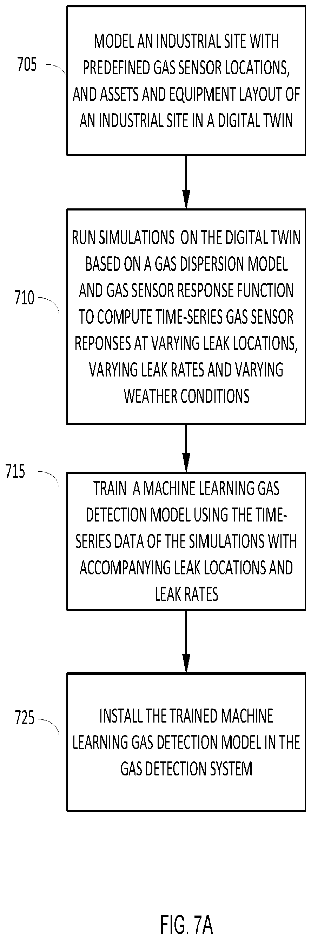

FIG. 7A diagrammatically illustrates the method of the present disclosure of training a machine learning gas detection model 210 to locate and/or record gas leak rates. In step 705 the industrial site 100 where the machine learning gas detection model will be deployed is virtually modeled in a digital twin 200 using predefined gas sensor locations and the assets, layout and equipment located at the industrial site 100.

In step 710 the digital twin 200 uses the predefined gas sensor locations and the modeled layout of the site and its equipment runs simulations using the gas dispersion model 225 and the gas sensor response model 235. The simulations executed by the digital twin 200 compute time-series gas sensor responses at varying simulated gas leak locations at varying gas leak rates using varying wind patterns of wind speeds and directions.

In step 715 the machine learning model gas detection model 210 is trained with the time-series responses and data run by the simulations. Along with the time-series gas sensor responses the machine learning model receives the gas leak locations and gas leak rates of the gas leaks used in the simulation run by the digital twin 200.

The machine learning gas detection model 210 is then transferred to the physical gas detection system 175 at the industrial site 100. A copy of the machine learning model may be left behind in the digital twin 200, to aid in retraining the machine learning gas detection model in a later continuous-learning method that retrains and tunes the machine learning gas detection model with data from the physical industrial site.

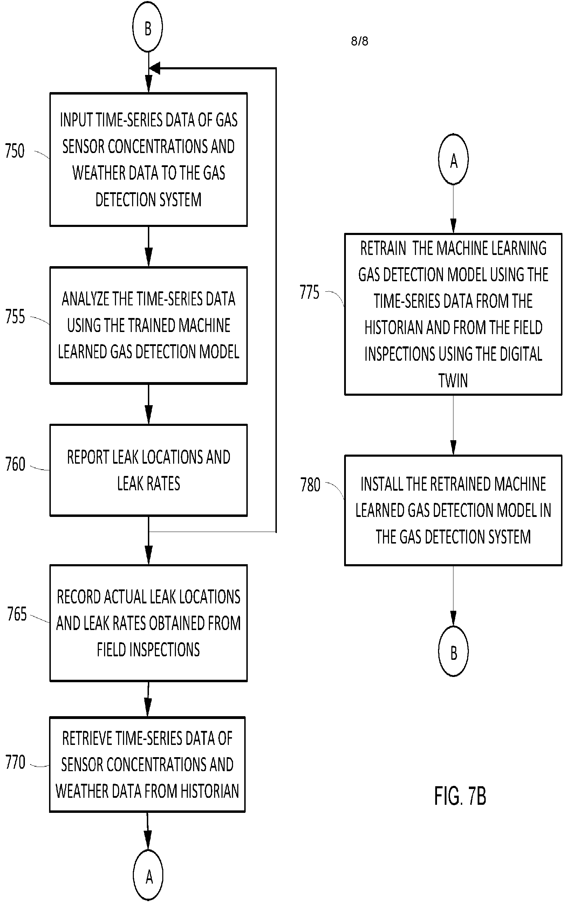

FIG. 7B diagrammatically illustrate the method in which the now machine learned gas detection model 211 is used with the gas detection system 175 of system 101. In step 750 the actual data gas concentrations from sensors 110 and weather data from the weather stations 120 at the industrial site are input as time-series data of gas sensor concentrations into the gas detection model 175 executing in the plant server 160.

In step 755, the time-series data of gas concentrations and weather data from the on-site weather stations 120 are input from the gas detection system 175 to the machine learned gas detection model 211 where the data is analyzed against the learned gas leak data from the training simulations. Suspected gas leaks and leak rates emanating from the equipment are then reported to the gas detection system 175 in step 760 for inspection by plant personnel. The operational process of steps 750-760 is repeated to continue the gas detection process. It should be noted that during the operational step 750 the time-series data of concentration from the sensors 110 and weather data from weather stations 120 are also historized in historian 170.

When an actual gas leak location and/or leak rate are obtained from a field inspection, a continuous learning process is used to tune the machine learned gas detection model 211 by retraining the machine learning model in the digital twin 200.

The actual gas leak and/or gas leak rate is recorded to the historian 170 in step 765. Next in step 770, the historic time-series data of gas concentrations and weather data, are retrieved and fed back to the digital twin 200 along with the data of the actual gas leak and/or leak rate from step 765 from the field inspections.

In step 775, the machine learning gas detection model 210 is retrained using the data from the steps 765 and 770 and the now retrained machine learned gas detection model 211 is loaded into the gas detection model 175 in step 780. The retraining and subsequent deployment of a retrained machine learned gas detection model is a continuous process triggered when actual leaks are discovered, thereby tuning the model to provide a more accurate detection of the gas leaks and/or leak rates at the industrial site.

It may be advantageous to set forth definitions of certain words and phrases used throughout this patent document. The term "communicate," as well as derivatives thereof, encompasses both direct and indirect communication. The terms "include" and "comprise," as well as derivatives thereof, mean inclusion without limitation. The term "or" is inclusive, meaning and/or. The phrase "associated with," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, have a relationship to or with, or the like. The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C.

The description in the present application should not be read as implying that any particular element, step, or function is an essential or critical element that must be included in the claim scope. The scope of patented subject matter is defined only by the allowed claims. Moreover, none of the claims is intended to invoke 35 U.S.C. § 112(f) with respect to any of the appended claims or claim elements unless the exact words "means for" or "step for" are explicitly used in the particular claim, followed by a participle phrase identifying a function. Use of terms such as (but not limited to) "mechanism," "module," "device," "unit," "component," "element," "member," "apparatus," "machine," "system," or "controller" within a claim is understood and intended to refer to structures known to those skilled in the relevant art, as further modified or enhanced by the features of the claims themselves and is not intended to invoke 35 U.S.C. §112(f).

While this disclosure has described certain embodiments and generally associated methods, alterations and permutations of these embodiments and methods will be apparent to those skilled in the art. Accordingly, the above description of example embodiments does not define or constrain this disclosure. Other changes, substitutions, and alterations are also possible without departing from the spirit and scope of this disclosure, as defined by the following claims.

## Claims

1. A system for training a machine learning gas detection model used in monitoring an
industrial site for gas leaks, comprising:
a gas sensor module containing data representing predefined locations of gas sensors located at the industrial site;
a gas sensor response model that models the responses of the gas sensors; and
a digital twin containing a virtual representation of the physical equipment located at the industrial site,
wherein the digital twin executes simulations of gas leaks using the virtual representation of the industrial site and varying simulated wind patterns, gas leak locations and leak rates including simulated gas sensor responses, to generate time-series gas sensor responses that are used to train the machine learning gas detection model for detecting gas leaks at the industrial site.

2. The system of Claim 1, wherein the virtual representations of the physical equipment located at the industrial site is data input into an industrial site model.

3. The system of Claim 1, wherein the system includes: predefined leak locations and leak rate data used by the digital twin to execute the gas leaks simulations.

4. The system of Claim 3, wherein the system includes: predefined weather data, used to simulate the varying simulated wind patterns used in the gas leak simulations.

5. The system of Claim 4, wherein the system includes: a gas dispersion model executed by the digital twin that receives the virtual representation of the industrial site and predefined data representing varying simulated wind patterns, gas leak locations and leak rates that generate estimated gas leak locations for the gas leak simulations.

6. The system of Claim 5, wherein the gas sensor response model receives the estimated locations for the simulated gas leaks from the gas dispersion model and generates the time-series gas sensor responses to train the machine learning gas detection model.

7. The system of Claim 1, wherein the system includes:
a training server executing the digital twin, the training server communicatively connected to a gas detection system; and
a plant server executing the gas detection system used for monitoring for gas leaks at the industrial site,
wherein the trained machine learning gas detection model is coupled to the plant server from the training server and used by the gas detection system to monitor the industrial site for gas leaks.

8. The system of Claim 7, wherein the gas detection system includes: a historian that collects actual time-series gas sensor data from gas sensors located at the industrial site and actual weather data from weather stations located at the industrial site and stores the actual time-series gas sensor data and actual weather data to a historian database as historical data.

9. The system of Claim 8, wherein the historical data is input to the training server and the digital twin to retrain the trained machine learning gas detection model.

10. A method for training a machine learning gas detection model used in monitoring an
industrial site for gas leaks, the method comprising:
providing data representing the locations of gas sensors located at the industrial site;
providing a gas sensor response model that models the responses of the gas sensors;
providing a digital twin containing a virtual representation of the physical equipment located at the industrial site;
executing by the digital twin simulations of gas leaks using varying simulated wind patterns and
varying gas leak locations and leak rates and the data representing the gas sensor locations to generate simulated time-series gas sensor responses; and
training the machine learning gas detection model with the gas sensor responses.

11. The method of Claim 10, wherein the method includes:
providing data representing predefined leak locations and leak rate data used in generating the gas leak simulations.

12. The method of Claim 11, wherein the method includes:
providing predefined weather data, used to simulate the varying simulated wind patterns for the gas leak simulations.

13. The method of Claim 12, wherein the method includes:
providing a gas dispersion model executed by the digital twin that receives the virtual representation of the industrial site and predefined data representing varying simulated wind patterns, gas leak locations and leak rates that generate estimated gas leak locations for the gas leak simulations.

14. The method of Claim 13, wherein the gas sensor response model receives the estimated locations for the simulated gas leaks from the gas dispersion model and generates the time-series gas sensor responses to train the machine learning gas detection model.

15. The method of Claim 10, wherein the trained machine learning gas detection model is retrained using historical data from a historian that collects actual time-series gas sensor and weather data from gas sensors and weather stations located at the industrial site.
